# EUROPEAN PATENT APPLICATION

(11) **EP 2 796 440 A1**
(43) Date of publication of application: **29.10.2014**
(21) Application number: 13842119.3
(22) Date of filing: 26.09.2013
(51) Int. Cl.: C07C 29/76, C07C 31/24, B01D 15/08

(54) **APPARATUS AND METHOD FOR PREPARING ALCOHOL FROM OLEFIN**

(30) Priority: 28.09.2012 KR 20120109128
(71) Applicant: LG Chem, Ltd., Seoul 150-721 (KR)
(72) Inventor: EOM, Sung Shik, Daejeon 305-738 (KR); KO, Dong Hyun, Daejeon 305-738 (KR); KIM, Dae Chul, Daejeon 305-738 (KR)
(74) Representative: Goddar, Heinz J.
(86) International application number: PCT/KR2013/008618
(87) International publication number: WO 2014/051347

(57) **Abstract**

Disclosed are an apparatus and method for preparing alcohol from olefin. A reactor for hydroformylating olefin comprises a loop reactor for reducing high-boiling point components, a post-treatment device for separating aldehyde comprises a catalyst/aldehyde separator and a divided wall column (DWC) for removing remaining high-boiling point components, and a post-treatment device for separating alcohol comprises a divided wall column (DWC) for removing remaining high-boiling point components. The apparatus and method for preparing alcohol reduce production of high-boiling point components in the preparation of alcohols and efficiently remove remaining high-boiling point components, thus obtaining alcohol containing no high-boiling point components.

## Description

### [Technical Field]

The present invention relates to an apparatus and method for preparing alcohol from olefin. More specifically, the present invention relates to an apparatus and method for preparing alcohol which reduce production of high-boiling point components in the preparation of alcohols and efficiently remove remaining high-boiling point components to obtain alcohol containing no high-boiling point component.

### [Background Art]

Hydroformylation well-known as an "oxo reaction" is a process of producing linear (normal) and branched (iso) aldehydes having one more carbon than olefins by reacting various olefins with synthetic gas (CO/H₂) in the presence of a metallic catalyst and a ligand.

Aldehydes synthesized by the oxo reaction are converted into aldehyde derivatives, i.e., acids and alcohols, via oxidation or reduction. Furthermore, aldehydes may be converted into acids and alcohols containing a long alkyl group via aldol condensation or the like and then oxidation or reduction. These alcohols and acids are used for solvents, additives, materials for various plasticizers and the like.

A representative example of hydroformylation is preparation of octanol(2-ethylhexanol) from propylene using a rhodium-based catalyst. Octanol is primarily used as a raw material for PVC plasticizers such as dioctyl phthalate (DOP) and is also used as intermediate raw materials for synthetic lubricants, surfactants and the like.

For example, in a case in which propylene is used as olefin, the propylene is injected together with synthetic gas (CO/H₂) into an oxo reactor using a catalyst to produce normal-butyraldehyde and iso-butyraldehyde. The produced aldehyde mixture is transferred together with a catalyst mixture to a separator and is separated into hydrocarbon and the catalyst mixture, the catalyst mixture is returned to the reactor and the hydrocarbon component is transferred to a stripper. The hydrocarbon is stripped in the stripper by fresh synthetic gas, the unreacted olefin and synthetic gas are recovered to the oxo reactor, and the butyraldehyde is transferred to a distillation column and is separated into normal- and iso-butyraldehyde. The normal-butyraldehyde present in the bottom of the distillation column is transferred to a hydrogenation reactor and is produced into n-butanol by hydrogenation (addition of hydrogen).

The normal-butyraldehyde present in the bottom of the distillation column primarily contains high-boiling point components such as aldehyde dimers or trimers produced during hydroformylation. When the normal-butyraldehyde containing high-boiling point components is supplied to the hydrogenation reactor and is then subjected to hydrogenation, problems of low product yield and decreased catalyst activity are generated. In order to solve these problems, the high-boiling point component is further purified using an additional column before transferred to the hydrogenation reactor.

Furthermore, hydrogenation of aldehyde is commonly performed using a reactor packed with a nickel-based or copper-based solid hydrogenation catalyst. The hydrogenation is carried out in vapor by evaporating starting aldehyde or in liquid by injecting a liquid-phase starting aldehyde into a reactor.

However, in spite of the catalysts and vapor- or liquid-phase reaction, undesired side reactions such as esterification, acetalization and esterification are generated during the reactions, thus disadvantageously causing deterioration in reaction selectivity and requiring additional purification of high-boiling point components incorporated in alcohol in the process of separating the produced alcohol.

### [Disclosure]

### [Technical Problem]

The inventors of the present invention designed and completed an apparatus and method for preparing alcohol which reduce generation of high-boiling point components during hydrogenation and remove remaining high-boiling point components by an alcohol separation process, based on the fact that necessity of further purification or deterioration in catalyst efficiency are caused by high-boiling point components generated during hydrogenation.

In accordance with one aspect of the present invention, provided is an apparatus for preparing alcohol comprising a reactor for hydroformylating olefin, a post-treatment device for separating aldehyde, a hydrogenation reactor and a post-treatment device for separating alcohol, wherein the reactor for hydroformylating olefin comprises a loop reactor for reducing high-boiling point components, the post-treatment device for separating aldehyde comprises a catalyst/aldehyde separator and a divided wall column (DWC) for removing remaining high-boiling point components, and the post-treatment device for separating alcohol comprises a divided wall column (DWC) for removing remaining high-boiling point components.

In accordance with another aspect of the present invention, provided is a method for preparing alcohol comprising hydroformylation of olefin, post-treatment for separating aldehyde, hydrogenation and post-treatment for separating alcohol, wherein the hydroformylation of olefin comprises reducing high-boiling point components, the post-treatment for separating aldehyde comprises recirculating a catalyst solution to the hydroformylation by gas-liquid separation, and the post-treatment for separating alcohol comprises removing remaining high-boiling point components by distillation.

Hereinafter, the method will be described in detail.

First, the present invention provides an apparatus and method for preparing alcohol to reduce high-boiling point components during hydrogenation and remove remaining high-boiling point components during separation of alcohol.

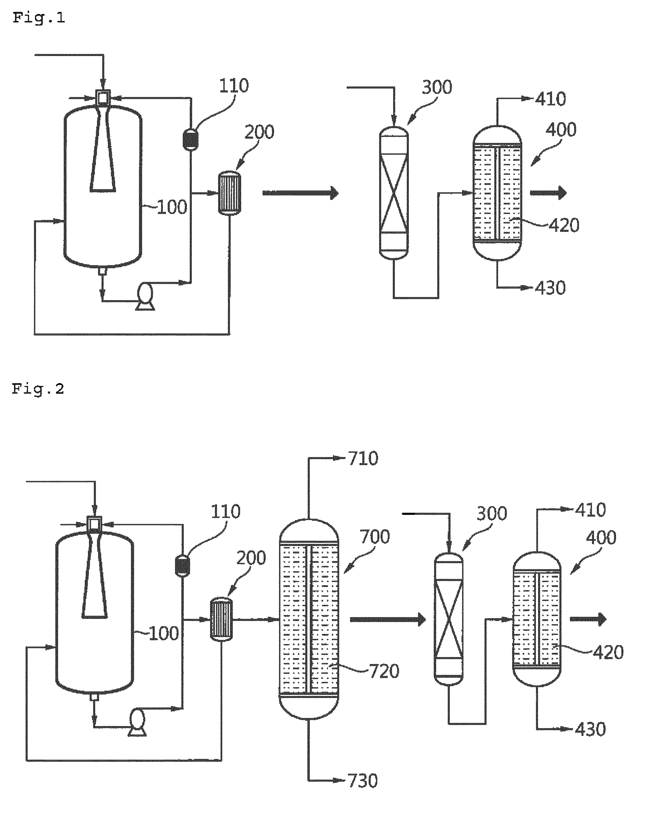

Hereinafter, a system for preparing alcohol from olefin according to an embodiment of the present invention will be described in detail with reference to the annexed drawings. FIG. 1 is a schematic view illustrating the system for preparing alcohol from olefin according to the embodiment.

Specifically, the system for preparing alcohol according to the present invention comprises a reactor for hydroformylating olefin, a post-treatment device for separating aldehyde, a hydrogenation reactor and a post-treatment device for separating alcohol, wherein the reactor for hydroformylating olefin comprises a loop reactor 100 for reducing high-boiling point components, the post-treatment device for separating aldehyde comprises a catalyst/aldehyde separator 200 and a divided wall column (DWC) 700 for removing remaining high-boiling point components, and the post-treatment device for separating alcohol comprises a divided wall column (DWC) 400 for removing remaining high-boiling point components.

Hereinafter, the loop reactor will be described in more detail.

Olefin and synthetic gas are sprayed to a catalyst mixture solution charged in the reactor by a spray mounted at the top of the loop reactor.

There is no particular limitation as to the spray so long as it is capable of spraying the olefin and synthetic gas to a catalyst mixture solution charged in the reactor. For example, the spray is an ejector equipped with a nozzle. The nozzle mounted on the ejector reduces a spray cross-section area of olefin and synthetic gas supplied into the reactor at a high pressure, thereby increasing a spray rate. A diameter of the nozzle depends on the size of the reactor and generally, is preferably 0.1 to 500 mm.

In addition, a venturi tube is preferably coupled to the ejector. The venturi tube includes an inlet which is a straight tube and a diffusion unit which is a tube having a bottom larger than a top, wherein the inlet is coupled to the ejector and a diameter of the inlet is the same as a diameter of an inlet of the diffusion unit and is smaller than a diameter of an outlet of the diffusion unit. In addition, the outlet of the diffusion unit preferably faces toward the bottom of the reactor. Preferably, the diameter of the inlet is preferably 0.2 to 1,000 mm and a length of the inlet of the diffusion unit is 1/50 to 1/2 the total length of the reactor. The diameter of the inlet of the diffusion unit is the same as that of the inlet and the diameter of the outlet of the diffusion unit is 1.0 to 10 times the diameter of the diffusion unit. In addition, the length of the diffusion unit is preferably 0.1 to 10 times the length of the inlet, and a total length of the venturi tube including the inlet and the diffusion unit is preferably 0.01 to 0.95 times the length of the reactor body, more preferably 0.05 to 0.75 times.

Reaction materials, *i.e.,* olefin and mixed gas are sprayed into the reactor while passing through the ejector and the venturi tube coupled thereto. The sprayed olefin and mixed gas form fine foams and at the same time are sprayed into the catalyst mixture solution charged in the reactor. The fine foams of the olefin and mix gas contact the catalyst mixture solution, thus increasing a gas-liquid contact surface area and providing a sufficient reaction area.

When the olefin and synthetic gas are sprayed into the catalyst mixture solution, hydroformylation proceeds and a reaction mixture comprising the aldehyde, the catalyst mixture solution, unconverted olefin, synthetic gas and other reaction by-products is present in the reactor. The reaction mixture is recovered from the bottom of the reactor by the reactor outlet and a circulation tube connected to the spray and is supplied to the spray provided on the top of the reactor. Such circulation enables the reaction mixture to be sufficiently sprayed together with reaction raw materials and mixed therewith and thereby improves reaction efficiency. This circulation can be controlled by a circulation pump provided in the circulation tube.

In addition, the circulation tube may be provided with a heat exchanger 110 and the position thereof is not particularly limited so long as it is disposed in a portion of the circulation tube. The heat exchanger 110 functions to maintain the reaction mixture circulated to the reactor at a temperature suitable for hydroformylation reaction conditions.

The reaction mixture branching from a portion of the circulation tube connected to the hydroformylation reactor is separated into a catalyst mixture solution and aldehyde by the catalyst/aldehyde separator 200, the catalyst mixture solution is circulated to the reactor 100 and the aldehyde is transferred to the hydrogenation reactor 300.

More specifically, the catalyst/aldehyde separator 200 comprises a separation tube branching from any portion of the circulation tube connected to the hydroformylation reactor 100 and separating the reaction mixture from a circulation flow, a catalyst/aldehyde separator 200 connected to the separation tube and separating a catalyst mixture solution and aldehyde from the reaction mixture, and a catalyst mixture solution supply tube connected to a portion of the catalyst/aldehyde separator and supplying the catalyst mixture solution to the circulation tube.

That is, the reaction mixture of the hydroformylation reactor 100 is supplied to the catalyst/aldehyde separator 200, and the catalyst mixture solution separated from the catalyst/aldehyde separator 200 is circulated to the hydroformylation reactor 100 through the catalyst mixture solution supply tube connected to the any portion of the circulation tube. There is no limitation as to the type of the catalyst/aldehyde separator 200 so long as the catalyst/aldehyde separator 200 is capable of separating the catalyst mixture solution and aldehyde from the reaction mixture. For example, the catalyst/aldehyde separator 200 is a vaporizer as a gas-liquid separator that discharges aldehyde, which is a low-boiling point component of the reaction mixture, in a vapor form and a catalyst mixture solution, which is a high-boiling point component, in a liquid form.

Recirculation of the catalyst mixture solution free of aldehyde may be continuously carried out. Where necessary, a part of the circulated reaction mixture is discharged to reproduce a catalyst and a new catalyst solution or a re-activated catalyst solution is added to a circulation flow of the reaction mixture.

The separated aldehyde is directly supplied as a mixture of the high-boiling point component, iso-type aldehyde and normal-type aldehyde into the hydrogenation reactor 300, or is supplied to a distillation column 700 connected to the catalyst/aldehyde separator 200 and separating aldehyde which is then supplied to the hydrogenation reactor 300.

The distillation column 700 is preferably a divided wall column (DWC) 700 which is capable of separating iso-type aldehyde, normal-type aldehyde and a high-boiling point component. Specifically, the divided wall column 700 includes an inlet a low-boiling point component outlet 710, a middle-boiling point component outlet 720 and a high-boiling point component outlet 730, wherein the respective outlets are divided by a division wall designed to be insulated and temperature and pressure of the inlet and the outlets 710, 720 and 730 are independently controlled.

The inlet is preferably operated at 20 to 100°C and at a pressure of 1.0 to 5.0 bar. In the inlet, the low-boiling point components, i.e., water and iso-type aldehyde, among the hydrogenation products are vaporized and are transferred to the low-boiling point component outlet 710 and is discharged through the low-boiling point discharge tube The low-boiling point component outlet 710 is preferably operated at 30 to 120°C and at a pressure of 0.1 to 5.0 bar. The middle-boiling point component, which is not vaporized in the inlet and the low-boiling point component outlet 710, is transferred to the middle-boiling point component outlet 720 and is then discharged through the middle-boiling point component discharge tube. A major ingredient of the middle-boiling point component is normal-type aldehyde. The middle-boiling point component outlet 720 is preferably operated at 40 to 170°C and at a pressure of 0.01 to 5.0 bar. In addition, the high-boiling point component which is not vaporized in the middle-boiling point component outlet is transferred to the high-boiling point component outlet 730 and is then discharged through the high boiling point component discharge tube. The high-boiling point components among the reaction products include an aldehyde dimer, an aldehyde trimer and the like. The high-boiling point component outlet 730 is preferably operated at 60 to 250°C and at a pressure of 0.1 to 5.0 bar.

Furthermore, in addition to the catalyst/aldehyde separator 200, the system for preparing alcohol from olefin may further include an aldol condensation reactor for preparing aldehyde having increased carbon atoms by aldol condensation of the normal-aldehyde.

When the aldol condensation reactor is further provided, alcohol having 2 times more carbon atoms than the normal-aldehyde can be produced. For example, when hydroformylation is performed using propylene, normal-butyraldehyde and iso-butyraldehyde are produced and 2-ethyl hexanal is produced by aldol condensation.

Aldehyde separated by the catalyst/aldehyde separator 200 is transferred to the hydrogenation reactor 300 and is converted into alcohol by hydrogenation.

The hydrogenation reactor 300 includes a spray for spraying the recovered aldehyde and hydrogen gas into a catalyst mixture solution charged in the reactor 300, a reactor outlet disposed in a lower part of the reactor and discharging a mixture of the aldehyde, the hydrogen gas and a product obtained by hydrogenation of aldehyde, and a circulation tube connected to the reactor outlet and the spray, recovering the mixture of the aldehyde, the hydrogen gas and a product obtained by hydrogenation of aldehyde from the reactor outlet and supplying the same to the spray.

The hydrogenation reactor 300 may be a catalyst reactor containing a hydrogenation catalyst in the form of a fixed bed.

The hydrogenation reactor 300 includes a spray for spraying the recovered aldehyde and hydrogen gas into the reactor 300, a nickel catalyst layer disposed around an inlet through which aldehyde and hydrogen are injected and performing hydrogenation and a reactor outlet disposed in a lower part of the reactor and discharging a hydrogenation mixture.

The aldehyde and hydrogen gas sprayed into the reactor pass through two catalyst layers and at the same time, produce hydrogenation products, i.e., alcohol. The hydrogenation products containing alcohol passing through the hydrogenation reactor 300 are transferred to a distillation column 400 for fractional distillation.

The distillation column 400 includes an inlet through which the hydrogenation products passing through the hydrogenation reactor 300 are injected, a low-boiling point component outlet 410 for discharging a low-boiling point component among the hydrogenation products, a middle-boiling point component outlet 420 for discharging a middle-boiling point component among the hydrogenation products and a high-boiling point component outlet 430 for discharging a high-boiling point component among the hydrogenation products.

The inlet and the respective outlets of the distillation column are divided by a division wall and the division wall is designed to be insulated so that temperature and pressure of the inlet and the outlets are independently controlled. The hydrogenation products having passed through the hydrogenation reactor contains alcohol, aldehyde, hydrogen, reaction by-product and the respective materials are fractionally distilled according to boiling point thereof.

The inlet is preferably operated at 20 to 100°C and at a pressure of 1.0 to 5.0 bar. Normal/iso-aldehyde, water, iso-alcohol and the like, which are low-boiling point components among the hydrogenation products are vaporized in the inlet, are transferred to the low-boiling point component outlet 410 and are discharged through a low-boiling point component discharge tube. The low-boiling point component outlet 410 is preferably operated at 30 to 120°C and at a pressure of 0.1 to 5.0 bar. The middle-boiling point component which is not vaporized in the inlet and the low-boiling point component outlet 410 is transferred to the middle-boiling point component outlet 420 and is then discharged through a middle-boiling point component discharge tube. The major component of the middle-boiling point components in the hydrogenation product is a mixture of normal-alcohol and iso-alcohol. The middle-boiling point component outlet 420 is preferably operated at 40 to 170°C and at a pressure of 0.01 to 5.0 bar. In addition, the high-boiling point component which is not vaporized in the middle-boiling point component outlet is transferred to the high-boiling point component outlet 430 and is discharged through a high-boiling point component discharge tube. The high-boiling point components among the hydrogenation products include normal-alcohol, an aldehyde dimer, an aldehyde trimer and the like. The high-boiling point component outlet 430 is preferably operated at 60 to 250°C and at a pressure of 0.1 to 5.0 bar.

A method for preparing alcohol using the device described above includes hydroformylation of olefin, post-treatment for separating aldehyde, hydrogenation and post-treatment for separating alcohol,

wherein the hydroformylation of olefin comprises reducing high-boiling point components, the post-treatment for separating aldehyde comprises re-circulating the catalyst solution to the hydroformylation by gas-liquid separation, and the post-treatment for separating alcohol comprises removing remaining high-boiling point components by distillation.

The post-treatment for separating aldehyde includes recirculating the catalyst solution to hydroformylation, distilling remaining aldehyde, and separating the high-boiling point component by the high-boiling point component outlet during distillation.

In addition, reduction of high-boiling point components during the hydroformylation of olefin is carried out by spraying olefin and synthetic gas (CO/H₂) into the catalyst mixture solution in the loop reactor to form fine foams of olefin and synthetic gas, and reacting the fine foams with the catalyst mixture solution while converting a spray flow of the olefin and synthetic gas.

The hydroformylation is a process of obtaining aldehyde by spraying olefin and synthetic gas (CO/H₂) into the catalyst mixture solution in the loop reactor to form fine foams of olefin and synthetic gas and reacting the fine foams with the catalyst mixture solution while converting a spray flow of the olefin and synthetic gas.

When olefin and synthetic gas are sprayed, fine foams are formed and contact the catalyst mixture solution, thus providing a sufficiently large reaction area due to wide vapor-liquid contact area. In addition, the reaction is performed while converting the spray flow of olefin and synthetic gas, thereby increasing a retention time of reaction materials in the reactor and improving reaction efficiency.

The hydroformylation is preferably carried out using the hydroformylation reactor described above.

The catalyst mixture solution of hydroformylation is any one generally used for hydroformylation and contains a transition metal catalyst and a ligand.

Any transition metal catalyst may be used without limitation so long as it is generally used in the art. For example, the transition metal catalyst is a catalyst containing a transition metal, such as cobalt (Co), rhodium (Rh), iridium (Ir), ruthenium (Ru), osmium (Os), platinum (Pt), palladium (Pd), iron (Fe), or nickel (Ni), as a center metal. Specifically, the transition metal catalyst comprises at least one complex catalyst selected from the group consisting of cobalt carbonyl [Co₂(CO)₈], acetylacetonato dicarbonyl rhodium [Rh(AcAc)(CO)₂], acetylacetonato carbonyl triphenylphosphine rhodium [Rh(AcAc)(CO)(TPP)], hydridocarbonyltri(triphenylphosphine)rhodium [HRh(CO) (TPP)₃], acetylacetonato dicarbonyl iridium [Ir(AcAc)(CO)₂] and hydridocarbonyl tri(triphenylphosphine)iridium [HIr(CO)(TPP)₃.

In addition, the ligand is trisubstituted phosphine, phosphine oxide, amine, amide, isonitrile or the like and is preferably trisubstituted phosphine. Examples of the trisubstituted phosphine include, but are not limited to, triaryl phosphine, triaryl phosphite, alkyl diaryl phosphine and the like. More specifically, the trisubstituted phosphine is triphenyl phosphine, tritolyl phosphine, triphenyl phosphite, n-butyl diphenyl phosphine or the like.

Examples of a solvent used for the catalyst mixture solution include, but are not limited to, aldehydes such as propane aldehyde, butyraldehyde, pentyl aldehyde, valeraldehyde or the like, ketones such as acetone, methyl ethyl ketone, methyl isobutyl ketone, acetophenone, cyclohexanone or the like, alcohols such as ethanol, pentanol, octanol, texanol or the like, aromatic compounds such as benzene, toluene, xylene or the like, ethers such as tetrahydrofuran, dimethoxyethane, dioxane or the like, and paraffin hydrocarbons such as heptanes. Preferably, the solvent is a reaction product such as propane aldehyde, butyraldehyde, pentyl aldehyde or valeraldehyde. In addition, a weight of the corresponding solvent present in the catalyst mixture solution is preferably 30% to 99% of the total solution weight.

The olefins that can be used in the present invention are C2 to C20 olefins, but the present invention is not limited thereto. More specifically, olefin is ethylene, propylene, 1-butene, 1-pentene, 1-hexene, 1-heptene, 1-octene, 1-nonene, 1-decene, 1-undecene, 1-tridecene, 1-tetradecene, 1-pentadecene, 1-hexadecene, 1-heptadecene, 1-octadecene, 1-nonadecene, 1-eicosene, 2-butene, 2-methylpropene, 2-pentene, 2-methylbutene, 2-hexene, 2-heptene, 2-ethyl hexene, 2-octene, styrene, 3-phenol-1-propene, 4-isopropylstyrene or the like. More preferably, olefin is ethylene, propylene, 1-butene, 2-butene, 1-pentene, 2-pentene, 2-methylbutane or the like.

The synthetic gas, which is another starting material of hydroformylation, is a mix gas of carbon monooxide and hydrogen and a mix ratio of CO to H₂ is preferably 5:95 to 70:30, more preferably, 40:60 to 60:40, most preferably 45:55 to 55:45, but the present invention is not limited thereto.

A molar ratio of the olefin to the synthetic gas is preferably 95:5 to 5:95, more preferably 75:25 to 25:75.

In addition, the olefin and synthetic gas are preferably each independently sprayed at a pressure of 5 to 200 bar. In addition, a linear velocity at which the olefin and synthetic gas are sprayed is preferably 1 m/sec to 50 m/sec, more preferably 5 m/sec to 30 m/sec. A difference in pressure between before and after the catalyst mixture solution passes through the spray is preferably 0.1 bar to 10 bar, more preferably 0.5 bar to 5 bar.

The reaction is preferably carried out at a temperature of 50 to 200°C, more preferably 50 to 150°C. In addition, the reaction is preferably carried out at a pressure of 5 to 100 bar, more preferably at a pressure of 5 to 50 bar.

In addition, preferably, the hydroformylation further includes circulating the reaction mixture including recovering the reaction mixture and supplying the same together with olefin and synthetic gas to the catalyst mixture solution.

The reaction mixture discharged through the reactor outlet is recovered and is sufficiently mixed with the reaction mixture by a circulation system supplied into the reactor, thus improving reaction efficiency. The reaction mixture contains, in addition to target substances, aldehydes (normal- and iso-butyraldehyde), unconverted olefin, reaction by-products, the catalyst mixture solution and the like.

Such a circulation system can be implemented by the reactor outlet, a circulation tube coupled to the spray of the reactor and a circulation pump coupled thereto. A flow of the circulated reaction mixture is preferably 0.01 to 20 times the amount of substances injected into the reactor per minute.

In addition, the hydroformylation further includes separating a portion of the circulated reaction mixture into a catalyst mixture solution and aldehyde, supplying the separated catalyst mixture solution to a circulation flow and recovering aldehyde.

Specifically, when olefin which is the starting material of hydroformylation is propylene, the reaction mixture contains butyraldehyde, more specifically, normal-butyraldehyde and iso-butyraldehyde, and the reaction mixture is transferred to the catalyst/aldehyde separator and is separated into aldehyde and the catalyst mixture, the catalyst mixture is circulated to the reactor and aldehyde is transferred for hydrogenation.

In the hydrogenation, a hydrogenation product containing alcohol is obtained by adding hydrogen to a product of hydroformylation, i.e., aldehyde. The hydrogenation of aldehyde is carried out by a method commonly used in the art and is preferably carried out by passing a highly active Ni catalyst layer through the recovered aldehyde and hydrogen gas.

The aldehyde of hydrogenation is obtained by hydroformylation of olefin and preferably contains 1 to 20 carbon atoms and one or more aldehyde groups, but is not limited thereto. Examples of the aldehyde include formaldehyde, acetaldehyde, propionaldehyde, n-butyraldehyde, iso-butyraldehyde, n-valeraldehyde, iso-valeraldehyde, n-hexaaldehyde, n-heptaaldehyde, n-octanal, 2-ethylhexanal, 2-ethylhexenal, n-decanal, 2-ethylbutanal, propargyl aldehyde, acrolein, glyoxal, crotonaldehyde, furfural, aldol, hexahydrobenzaldehyde, alpha-citronellalal, citral, chloral, trimethyl acetaldehyde, diethyl acetaldehyde, tetrahydrofurfural, phenyl aldehyde, cinnamaldehyde, hydro-cinnamaldehyde and the like. Preferably, the aldehyde is propionaldehyde, n-butyraldehyde, iso-butyraldehyde, n-valeraldehyde or iso-valeraldehyde.

For example, when hydroformylation is performed using propylene, normal-butyraldehyde and iso-butyraldehyde are produced and normal-butylalcohol and iso-butylalcohol are produce by hydrogenation.

The aldehyde is preferably sprayed at a rate of 0.1 to 100 m/sec. When aldehyde is sprayed at a constant rate, the hydrogen gas is suctioned into the hydrogenation reactor.

A molar ratio of aldehyde and hydrogen gas is preferably 1:10 to 10:1. Reaction temperature is 50 to 300°C and reaction pressure is 2 to 100 bar.

During the separation, structural isomers of alcohol are separated by fractional distillation of the hydrogenation product.

The hydrogenation product contains, in addition to a target substance, i.e., alcohol, aldehyde, hydrogen, reaction by-products and the like. The separation of the target substance, alcohol is carried out by a method commonly used in the art and is preferably carried out using the following method.

The separation of the hydrogenation product is carried out using a column having divided areas divided by a division wall. The division wall is designed to be insulated, and operation temperature and pressure thereof may be different from the operation temperature and pressure of a column commonly used according to position and structure of the division areas, and can be also suitably controlled according to design. The hydrogenation products are fractionally distilled according to boiling point while passing through the respective division areas. The low-boiling point components, i.e., normal- and iso-aldehyde, water, iso-alcohol, and the like among the hydrogenation products, are vaporized in the division area controlled to a relatively low temperature and pressure and are then discharged to an upper part of the column. In addition, the middle-boiling point components, i.e., iso-alcohol and normal-alcohol are not vaporized or are liquefied during vaporization and are then discharged from the middle-boiling point component area of the column. In addition, a small amount of high-boiling point components such as normal-alcohol, aldehyde dimer and aldehyde trimer are not vaporized and are discharged in a liquid form through the lower part of the column.

For example, when hydroformylation is performed using propylene, normal-butyraldehyde and iso-butyraldehyde are produced and are subjected to hydrogenation and distillation purification, to obtain normal-butyl alcohol and iso-butyl alcohol as final products.

The post-treatment for separating aldehyde further includes separating aldehyde obtained by gas-liquid separation into normal-aldehyde and iso-aldehyde by distillation and subjecting the normal-aldehyde to aldol condensation to obtain aldehyde having an increased number of carbon atoms and thereby perform hydrogenation using aldehyde having an increased number of carbon atoms. For example, when hydroformylation is performed using propylene, normal-butyraldehyde and iso-butyraldehyde are produced and 2-ethyl hexanal is produced by aldol condensation, Octanol(2-ethylhexanol) can be produced by hydrogenation using aldehyde having an increased number of carbon atoms. An aldol condensation reactor that can be used for the aldol condensation is a continuous stirred tank reactor (CSTR) or the like, but the present invention is not limited thereto.

The distillation is carried out by supplying the aldehyde containing a gas-phase high-boiling point component to an inlet of a divided wall column (DWC), and separating iso-type aldehyde in the low-boiling point component outlet, separating normal-type aldehyde in the middle-boiling point component outlet and separating the high-boiling point component in the high-boiling point component outlet.

### [Advantageous effects]

The present invention provides an apparatus and method for preparing alcohol which advantageously reduce production of high-boiling point components in the preparation of alcohols and efficiently remove remaining high-boiling point components to obtain alcohol containing no high-boiling point components.

### [Brief Description of the drawings]

FIGS. 1 to 3 are schematic views illustrating a process of preparing alcohol according to an embodiment of the present invention while reducing production of high-boiling point components and removing produced high-boiling point components.

### <The description of reference numerals of the main elements>

100: Hydroformylation reactor
110: Heat exchanger
200: Catalyst/aldehyde separator
300, 310: Hydrogenation reactors
400, 600, 700: Divided wall column
500: Aldol condensation reactor
410, 420, 430, 610, 620, 630, 710, 720, 730: Outlet

### [Best Mode]

Now, the present invention will be described in more detail with reference to the examples and comparative examples. These examples are only provided to illustrate the present invention and should not be construed as limiting the scope and spirit of the present invention.

### [Example]

### <Example 1>

### (Example 1.1.) Production of catalyst solution

3.2 kg of triphenyl phosphine (TPP) was completely dissolved in 28.7 kg of normal-butyraldehyde having a purity of 99%. 45.9 g of an acetylacetonatodicarbonyl triphenylphosphine rhodium (ROPAC)) catalyst which had been previously weighted was further added to the solution to prepare 32 kg of a catalyst solution.

### (Example 1.2) Preparation of aldehyde and gas-liquid separation

Referring to FIG. 1, two 30L loop reactors 100 were produced and a venturi diffusion tube having a nozzle diameter of 5 mm, a diffusion tube inlet diameter of 10 mm, a diffusion tube outlet diameter of 20 mm and a diffusion tube length of 30 mm was mounted on a head of each reactor. In addition, a flat diffusion plate having a diameter of 70 mm was fixed at a position of 200 mm from the lower outlet in the reactor. A circulation pump was mounted in the reactor such that a reaction solution could be recirculated at a flow rate of 20 L/min to a spray nozzle of each reactor head and a heat exchanger 110 was mounted on an external circulation line of both reactors so that reaction heat generated during reaction could be removed.

The two reactors were connected in series and, for a first reactor, which was a first reactor of the two reactors connected in series, a portion of a circulation line was connected to an upper part of a second reactor and a controller was mounted such that the first reactor could be continuously operated at a constant liquid level.

Like the first reactor, for the second reactor, which was a second reactor connected to the first reactor in series, a controller was mounted such that the second reactor could be continuously operated at a constant liquid level by supplying the reaction mixture from a portion of the circulation line to an evaporator for separating aldehyde.

The respective loop reactors connected in series were designed to independently supply propylene and synthetic gas, as feed materials. As reaction proceeded, aldehyde was recovered in a vaporizer 200 from the second reactor and the remaining reaction catalyst solution passed through an additional pump and was then recirculated to the first reactor. 16 kg of the previously prepared catalyst solution was charged in each of the two reactors, the reactor was purged twice with nitrogen gas and propylene, and reaction temperature was maintained at 89°C through a circulation pump and a heat exchanger. When the inner temperature of the reactor was stabilized, propylene was fed to the reactor until an inner pressure of each reactor reached 12 bar.

After the temperature and pressure were stabilized again, propylene as a raw material was fed to the first reactor at a flow rate of 3.7 kg/hr and synthetic gas was fed at a mean flow rate of 2.2 kg/hr and 0.5 kg/hr to the first and second reactors, respectively. A liquid level of each reactor was maintained at 20 liter, the pressure and temperature of the first reactor were stabilized and reached normal levels of 18 barg and 89°C, and the pressure and temperature of the second reactor were stabilized and reached normal levels of 15 barg and 89°C, and continuous operation was then performed for 240 hours.

As a result of analysis of a condensed component from the vaporizer 200 and measurement of an amount of produced butyraldehyde, 1,512 kg in total of butyraldehyde was produced, 6.3 kg of butyraldehyde on average was produced per hour, and propylene conversion efficiency, which means a level at which fed propylene is converted into butyraldehyde, rather than propane as a by-product, was 97.0%.

As a result of analysis of the obtained butyraldehyde product by gas chromatography, a content of butyraldehyde was 99.5%, a content of a low-boiling point component was 0.1% and a high-boiling point component including a phosphine compound was 0.4%.

### (Example 1.3) Hydrogenation of aldehyde using fixed bed catalyst reactor

An alumina ball was charged to a position of 57 cm from an upper part of a column-shaped reactor having a diameter of 8 cm and a length of 330 cm, as a hydrogenation reactor 300, a nickel catalyst supported on gamma-alumina was charged to a length of 315 cm therefrom, and the alumina ball was charged to the remaining lower part thereof. The temperature of the reactor outlet was maintained at a level not higher than 110°C using an additional circulation pump and an external heat exchanger and an inner pressure of the reactor was maintained at 25 barg.

Normal-butylalcohol was used as a solvent medium for reaction and heat exchange and a circulation flow was maintained at 38 kg/hr. Upon reaching a normal state, operation was performed for 90 hours and a total weight of a hydrogenation product containing butyl alcohol as a major component was 581 kg and the hydrogenation product was obtained in an amount of 6.45 kg per hour on average.

### (Example 1.4) Purification of alcohol using divided wall column (DWC)

An inner area of a pipe having a diameter of 8 cm and a length of 94 cm, excluding 10 cm of both ends of the pipe, was uniformly and vertically divided and blocked using a metal barrier, to produce a divided wall column 400. A pre-column into which a feed was injected was configured with a packed column having 18 theoretical plates, based on process simulation results, using a glass wool and a rashig ring having an average diameter of 1 cm and in the same manner, a main column facing a middle-boiling point component outlet was also configured with a packed column having 18 theoretical plates.

A packed column having 6 theoretical plates was configured in a lower part of a column equipped with a reboiler and in an upper part of a column equipped with a condenser in the same manner.

Accordingly, the pre-column provided around the feed inlet had 18 plates and the main column provided around the middle-boiling point component outlet has 30 plates in total. Through startup and stabilization, the hydrogenation product described in Example 1.3 was fed to the column at a flow rate of 6.4 kg/hr on the sixth plate of the pre-column, and the middle-boiling point component was continuously recovered on the eleventh plate from upper and lower parts of the column and the upper part of the main column.

On a weight basis, iso-butylalcohol and water as low-boiling point components discharged through the low-boiling point component outlet 410 of the column 400 were 8.6% and 0.2%, respectively, normal-butylalcohol discharged through the middle-boiling point component outlet 420 was 87.1% and a butyraldehyde trimer discharged through the high-boiling point component outlet 430 was 4.3%'.

### <Example 2>

With reference to FIG. 2, the same process as Example 1 was repeated except that aldehyde suggested in Example 1.2 was gas-liquid separated and was further purified using the same divided wall column (DWC) 700 as in Example 1.4 and separated normal butyraldehyde was fed to the same hydrogenation reactor as in Example 1.3.

Through startup and stabilization, the separated aldehyde gas-phase substance in Example 1.2 was fed at a flow rate of 6.4 kg/hr to the sixth plate of the pre-column, and the middle-boiling point component was continuously recovered on the twelfth plate from upper and lower parts of the column and the upper part of the main column. A total normal operation time was 86 hours and 1.07 kg of water in total and 20g or less of iso-butyraldehyde were obtained from the low-boiling point component outlet 710 of the upper part of the column. A three-component mixture containing 47.7 kg of iso-butyraldehyde, 476.6 kg of normal-butyraldehyde and 5g or less of water was obtained from the middle-boiling point component outlet 720 provided in a middle part of the column. In addition, 23 kg of an aldehyde trimer and 0.5 kg or less of normal-butyraldehyde were obtained from the high-boiling point component outlet 730 provided in the lower part of the column.

### <Example 3>

### (Example 3.1) Preparation of aldehyde having increased number of carbon atoms by aldol condensation

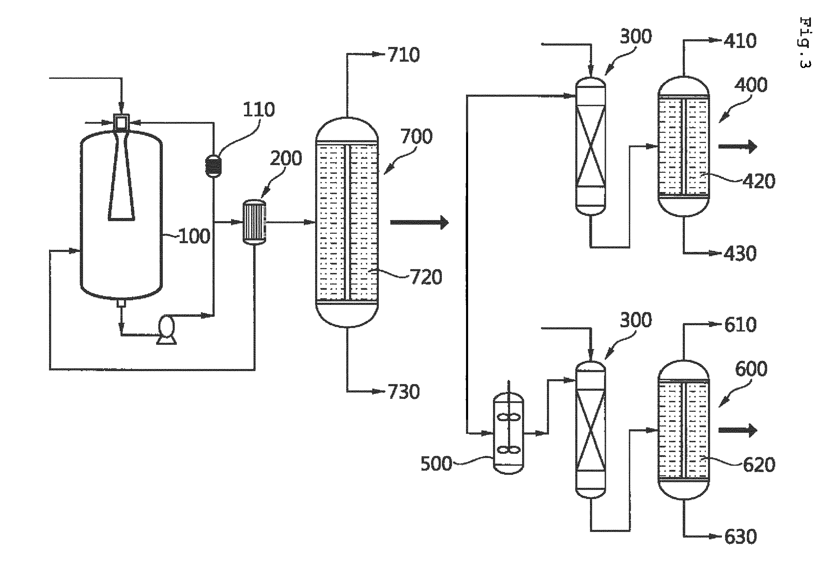

With reference to FIG. 3, 20L of a liquid containing a 2.0% aqueous NaOH solution and normal-butyraldehyde at a ratio of 1:2 was charged in a 30L vertical continuous stirred tank reactor (CSTR) 500 as an aldol condensation reactor 500 and an inner temperature and pressure of the reactor were maintained at 120°C and 5 barg, respectively.

The rate of revolutions was maintained at 300 rpm and the aldehyde mixture prepared in Example 1.3 was subjected to fractional distillation to obtain 240 kg of normal-butyraldehyde having a purity of 99% as a feed for aldol condensation reaction.

The normal-butyraldehyde thus obtained was continuously fed at a flow rate of 6.3 kg/hr, and a reaction product was recovered using a decanter under normal operation conditions for 32 hours while a liquid level was maintained at 20 liter.

A weight of the reaction product was 158 kg in total and as a result of analysis, ethyl propyl acrolein was 96%, normal-butyraldehyde was 3.9% and an aldehyde trimer was 0.1%, and on average, 74 kg of ethyl propyl acrolein was produced per hour.

### (Example 3.2) Preparation of alcohol having increased number of carbon atoms by hydrogenation

Hydrogenation was performed in the hydrogenation reactor 310 in the same manner as in Example 1.3 except that 96% of ethyl propyl acrolein, as the reaction product prepared in Example 3.1 was fed at an average flow rate of 4.7 kg/hr while feeding hydrogen at a flow rate of 0.26 kg/hr.

After reaching a normal state, continuous operation was performed for 28 hours at a constant liquid level. As a result, a total weight of a hydrogenation product containing octanol as a major component was 136 kg and the hydrogenation product was obtained in an amount of 4.86 kg per hour on average.

As a result of analysis using the same divided wall column 600 as in Example 1.4, on a weight basis, butanol and water as low-boiling point components discharged from the low-boiling point component outlet 610 were 0.5% and 0.2%, octanol discharged from the middle-boiling point component outlet 620 was 96% and a heavy component such as butyraldehyde trimer discharged from the high-boiling point component outlet 630 was 4.3%.

### <Comparative Example 1>

Continuous operation was performed in a normal state for 72 hours in the same manner as in Example 1 except that 30L two vertical continuous stirred tank reactors connected in series were used, instead of the loop reactor 100. As a result, 436 kg of butyraldehyde in total was produced, 6.06 kg of butyraldehyde on average was produced per hour and propylene conversion efficiency, which means a level at which fed propylene is converted into butyraldehyde, rather than propane as a by-product, was 95.6%.

As a result of analysis of the butyraldehyde product by gas chromatography, a content of butyraldehyde was 98.5%, a content of the low-boiling point component was 0.5% and the high-boiling point component including a phosphine compound was 1.0%.

Then, instead of the divided wall column 400 used in Example 1.4, two packed columns having 20 theoretical plates provided with a reboiler and a condenser were produced using a pipe having the same diameter and length as the divided wall column 400 and were connected in series. A product was recovered from a top while the same feed as in Example 1.3 was fed at a flow rate of 6.4 kg/hr on the eighth plate from the upper part of the first column, the product of the bottom was fed to the eighth plate from the upper part of the second column and a product was recovered from the top of the second column.

Total normal operation time was 70 hours and 0.89 kg of water in total and 15g or less of iso-butyraldehyde were obtained as top products of the first column. A three-component mixture containing 38.7 kg of iso-butyraldehyde, 388.3 kg of normal-butyraldehyde and 7g of water was obtained as a top product of the second column. 18.6 kg of an aldehyde trimer and 0.7 kg of normal-butyraldehyde were obtained as final bottom products.

## Claims

1. An apparatus for preparing alcohol comprising:
a reactor for hydroformylating olefin;
a post-treatment device for separating aldehyde;
a hydrogenation reactor; and
a post-treatment device for separating alcohol,
wherein the reactor for hydroformylating olefin comprises a loop reactor for reducing high-boiling point components, the post-treatment device for separating aldehyde comprises a catalyst/aldehyde separator and a divided wall column (DWC) for removing remaining high-boiling point components, and the post-treatment device for separating alcohol comprises a divided wall column (DWC) for removing remaining high-boiling point components.

2. The apparatus according to claim 1, wherein the catalyst/aldehyde separator for circulating a catalyst comprises a vaporizer for separating a gas-phase aldehyde and a liquid-phase catalyst solution.

3. The apparatus according to claim 1, wherein the divided wall column for removing the high-boiling point component comprises an inlet, a low-boiling point component outlet, a middle-boiling point component outlet and a high-boiling point component outlet,
wherein the respective outlets are divided by a division wall designed to be insulated and temperature and pressure of the inlet and the outlets are independently controlled.

4. The apparatus according to claim 3, wherein the inlet is operated at 20 to 100°C and 1.0 to 5.0 bar, the low-boiling point component outlet is operated at 30 to 1.20°C and 0.1 to 5.0 bar, the middle-boiling point component outlet is operated at 40 to 170°C and 0.01 to 5.0 bar, and the high-boiling point component outlet is operated at 60 to 250°C and 0.1 to 5.0 bar.

5. The apparatus according to claim 1, further comprising an aldol condensation reactor disposed between the post-treatment device for separating aldehyde and the hydrogenation reactor, the aldol condensation reactor performing aldol condensation of the normal-aldehyde to prepare aldehyde having an increased number of carbon atoms.

6. The apparatus according to claim 1, wherein the hydrogenation reactor comprises:
a spray for spraying aldehyde and hydrogen gas into the reactor;
a nickel catalyst layer disposed near a region where aldehyde and hydrogen are injected, the nickel catalyst layer performing hydrogenation; and
a reactor outlet disposed in a lower part of the reactor, the reactor outlet discharging a hydrogenation mixture.

7. A method for preparing alcohol comprising:
hydroformylation of olefin;
post-treatment for separating aldehyde;
hydrogenation; and
post-treatment for separating alcohol,
wherein the hydroformylation of olefin comprises reducing high-boiling point components, the post-treatment for separating aldehyde comprises recirculating a catalyst solution to the hydroformylation by gas-liquid separation, and the post-treatment for separating alcohol comprises removing remaining high-boiling point components.

8. The method according to claim 4, wherein the post-treatment for separating aldehyde comprises:
recirculating the catalyst solution to the hydroformylation by gas-liquid separation;
distilling remaining aldehyde; and
separating a high-boiling point component by a high-boiling point component outlet during distillation.

9. The method according to claim 7, wherein the reduction of high-boiling point components during the hydroformylation of olefin is carried out by spraying olefin and synthetic gas (CO/H₂) into the catalyst mixture solution in the loop reactor to form fine foams of olefin and synthetic gas and reacting the fine foams with the catalyst mixture solution while converting a spray flow of the olefin and synthetic gas.

10. The method according to claim 7, wherein the hydrogenation comprises liquid-reacting the aldehyde with hydrogen gas by passing a highly active Ni catalyst layer through the aldehyde and the hydrogen gas.

11. The method according to claim 10, wherein the aldehyde of the hydrogenation comprises at least one selected from the group consisting of formaldehyde, acetaldehyde, propionaldehyde, n-butyraldehyde, iso-butyraldehyde, n-valeraldehyde, iso-valeraldehyde, n-hexaaldehyde, n-heptaaldehyde, n-octanal, 2-ethylhexanal, 2-ethylhexenal, n-decanal, 2-ethylbutanal, propargyl aldehyde, acrolein, glyoxal, crotonaldehyde, furfural, aldol, hexahydrobenzaldehyde, alpha-citronellalal, citral, chloral, trimethyl acetaldehyde, diethyl acetaldehyde, tetrahydrofurfural, phenyl aldehyde, cinnamaldehyde and hydro-cinnamaldehyde.

12. The method according to claim 10, wherein the aldehyde is sprayed at a rate of 0.1 to 100 m/sec.

13. The method according to claim 7, wherein the post-treatment for separating aldehyde further comprises:
separating the aldehyde obtained by gas-liquid separation into normal-aldehyde and iso-aldehyde by distillation; and
subjecting the normal-aldehyde to aldol condensation to obtain aldehyde having an increased number of carbon atoms and thereby perform hydrogenation using aldehyde having an increased number of carbon atoms.

14. The method according to any one of claims 7, 8 and 13, wherein the distillation comprises:
supplying the gas-phase high-boiling point component-containing reaction product to the inlet of the divided wall column (DWC); and
separating the reaction product into a low-boiling point component, a middle-boiling point component and a high-boiling point component.

15. The method according to claim 7, wherein the olefin comprises at least one selected from the consisting of ethylene, propylene, butene, 1-hexene, 1-octene, 1-nonene, 1-decene, 1-undecene, 1-tridecene, 1-tetradecene, 1-pentadecene, 1-hexadecene, 1-heptadecene, 1-octadecene, 1-nonadecene, 1-eicosene, 2-butene, 2-methylpropene, 2-pentene, 2-hexene, 2-heptene, 2-ethyl hexene, 2-octene, styrene, 3-phenyl-1-propene and 4-isopropylstyrene.

16. The method according to claim 9, wherein the catalyst mixture solution comprises a transition metal catalyst, a ligand and a solvent.
